# EUROPEAN PATENT SPECIFICATION

(11) **EP 1 606 230 B1**
(45) Date of publication and mention of the grant of the patent: **19.09.2012**
(21) Application number: 04721749.2
(22) Date of filing: 18.03.2004
(51) Int. Cl.: C07C 41/16, C07C 41/26, C07C 43/178, C07D 317/20, C07D 317/34, C07C 319/06, C07D 319/06

(54) **PROCESS FOR MANUFACTURE OF AN ALLYL ETHER**
VERFAHREN ZUR HERSTELLUNG EINES ALLYLETHERS
PROCEDE DE PRODUCTION D'UN ETHER ALLYLIQUE

(30) Priority: 21.03.2003 SE 0300765
(43) Date of publication of application: 21.12.2005
(73) Proprietor: Perstorp Specialty Chemicals AB, 284 80 Perstorp (SE)
(72) Inventor: SAMUELSSON, Jesper, S-282 33 Tyringe (SE); PAJALIC, Oleg, S-291 66 Kristianstad (SE); MANEA, Mircea, S-280 22 Vittsjö (SE); OGEMARK, Keith, S-284 35 Perstorp (SE)
(74) Representative: HOFFMANN EITLE
(86) International application number: PCT/SE2004/000399
(87) International publication number: WO 2004/083153

(56) References cited:
- WO-A1-00/35895
- WO-A1-94/00539
- GB-A- 821 977
- GRAS J.L. ET AL: 'Transacetalisation de triols a partir du dimethoxymethane. Selectivite et applications synthetiques' TETRAHEDRON LETTERS vol. 28, no. 52, 1987, pages 6601 - 6604, XP002979287

## Description

The present invention relates to the production of an allyl and/or methallyl ether of a tri or polyhydric alcohol, such as a 2-alkyl-2-hydroxyalkyl-1,3-propanediol or a 2,2-dihydroxyalkyl--1,3-propanediol and/or a cyclic formal thereof. More specifically, the invention relates to a novel process for the preparation of an allyl and/or methallyl ether from a waste stream comprising at least one cyclic formal of a said tri or polyhydric alcohol.

Allyl and methallyl ethers of polyhydric alcohols are typically produced according to the Williamson synthesis by reacting a polyhydric alcohol with at least one allyl or methallyl halide, such as allyl bromide or methallyl chloride, in the presence of a basic catalyst. GB-A-821977 discloses such a process.

Tri and polyalcohols having for instance a neopentyl structure, such as trimethylolpropane, trimethylolethane and pentaerythritol are normally synthesised in an alkali catalysed aldolcondensation of formaldehyde and a second aldehyde. Yielded aldolaldehyde is subsequently reduced to corresponding alcohol, by means of a so called Cannizzaro reaction, with a further amount of formaldehyde in the presence of a strong base. The reaction can alternatively be carried out by means of catalytic hydration. The synthesis as well as recovery of obtained reaction product normally yield secondary products, such as formals (formaldehyde acetals). The syntheses yield linear and cyclic formals, such as 1,3-dioxolanes and 1,3-dioxanes. Linear formals are when exposed to acidic treatment, for instance when passing an ion exchanger, during recovery of the primary product transformed into cyclic formals.

Cyclic formals are also yielded during recovery if a synthesised tri or polyalcohol under acidic conditions is in contact with formaldehyde. This occurs for instance during an evaporation procedure, wherein water and excess of formaldehyde are evaporated. Formation of formals occurs when a 1,3-diol structure in a di, tri or polyalcohol reacts with for instance formaldehyde to corresponding 1,3-dioxane. Glycerol can similarly yield formals, such as 4-hydroxymethyl-1,3-dioxolane and 5-hydroxy-1,3-dioxane. Cyclic formals are furthermore yielded during other polyalcohol syntheses, such as acid catalysed etherification, as disclosed in for instance the US Patent 3,673,226.

Formals yielded as by-products in a synthesis of a polyhydric alcohol are continuously subjected to development efforts making said formals technically and commercially valuable. One such achievement is represented by 5-ethyl-5-hydroxymethyl-1,3-dioxane (cyclic trimethylolpropane formal) now frequently used for instance as flow additive in cement compositions and for preparation of reactive acrylic diluents.

The present invention quite unexpectedly provides a process wherein cyclic formals, for instance yielded as by-products in a synthesis of a tri or polyhydric alcohol, are converted to technically and commercially valuable products, such as allyl ethers of tri and polyhydric alcohols or allyl ethers of said cyclic formals. The present invention accordingly refers to a process for production of an allyl and/or methallyl ether of a tri or polyhydric alcohol.

The process of the present invention comprises the steps of
i) subjecting at least one cyclic formal of a tri or polyhydric alcohol to allylation by reaction with at least one allyl and/or methallyl halide in the presence of a catalytically effective amount of at least one basic catalyst, whereby a reaction mixture comprising at least one allyl and/or methallyl ether of said cyclic formal is yielded, and
ii) subjecting in step (i) obtained allyl and/or methallyl ether of said cyclic formal to reaction with at least one alcohol, having one or more hydroxyl groups, optionally in presence of a catalytically effective amount of at least one organic acid catalyst, whereby a reaction mixture comprising at least one allyl and/or methallyl ether of said tri or polyalcohol and at least one formal of said alcohol, is yielded
and optionally an intermediate purification step wherein the reaction mixture obtained in step (i) is purified prior to initiation of step (ii) and/or a final purification step wherein the reaction mixture obtained in step (ii) is purified.

Step (i) of said process is preferably performed at atmospheric or at a reduced or increased pressure and at a temperature of 60-140°C and step (ii) is likewise preferably performed at a reduced pressure, such as a pressure of less than 2000 Pa (15 mm Hg), or at a atmospheric or increased pressure and at a temperature of 80-160°C. Said allyl and/or methallyl halide and said basic catalyst are in step (i) advantageously added continuously to the reactor.

Said optional intermediate purification step comprises, in preferred embodiments of the process of the present invention, at least one extraction, such as mixing the reaction mixture obtained in step (i) with water and allowing obtained mixture to separate in a water phase and an organic phase and recovery of said organic phase. Said optional intermediate purification can also and optionally comprise further purifying, of for instance said organic phase, by for instance evaporation, such as distillation, at a pressure of for instance less than 2000 Pa (15 min Hg).

Said optional final purification step comprises, in preferred embodiments of the process of the present invention, purification of the reaction mixture obtained in step (ii) by for instance evaporation, such as distillation, at a pressure of for instance less than 2000 Pa (15 mm Hg). Said optional final purification may also comprise extraction as for instance disclosed above for said optional intermediate purification.

The cyclic formal subjected to allylation is suitably a recovered and optionally purified by-product or is present in a mixture, such as a waste stream which optionally is purified, of by-products from a plant wherein a tri or polyalcohol, such as glycerol, trimethylolethane, trimethylolpropane or pentaerythritol, is synthesised and produced.

Preferred embodiments of said cyclic formal include cyclic formals having a 1,3-dioxolane and most preferably a 1,3-dioxane structure, such as cyclic formals of 1,2,3-propanetriols, 2-alhyl-2-hydroxyalkyl-1,3-propanediols, 2-alkyl-2-hydroxyalkoxy-1,3 -propandiols, 2-alkyl--2-hydroxyalkoxyalkyl-1,3-propanediols, 2,2-dihydroxyalkyl-1,3-propanediols, 2,2-dihydroxyalkoxy-1,3-propanediols, 2,2-dihydroxyalkoxyalkyl-1,3-propanediols and dimers, trimers and polymers thereof. Said cyclic formal can suitably be exemplified by a cyclic formal of glycerol, trimethylolethane, trimethylolpropane, diglycerol, ditrimethylolethane, ditrimethylolpropane, pentaerythritol, dipentaerythritol and ethoxylated and/or propoxylated species thereof. Preferred embodiments of said cyclic formal include 4-hydroxyalkyl-1,3-dioxolanes, 5-hydroxy-1,3-dioxanes, 5-alkyl-5-hydroxy-1,3-dioxanes, 5-allcyl-5-hydroxyalkyl-1,3-dioxanes and/or 5,5-hydroxyalkyl-1,3-dioxanes. The most preferred cyclic formals are cyclic formals of tri or polyhydric alcohols having a neopentyl structure, such 2,2-substituted 1,3-propanediols. Especially preferred embodiments of the present invention include subjecting for instance 4-hydroxyalkyl-1,3-dioxolane, 5-hydroxy-1,3-dioxane, 5-alkyl-5-hydroxy-1,3-dioxane, 5-alkyl-5-hydroxyalkyl-1,3-dioxane and 5,5-hydroxyalkyl-1,3-dioxane to allylation in step (i) and subjecting obtained allyl and/or methallyl ether to reaction in step (ii).

Said at least one allyl and/or methallyl halide is, in embodiments of the present invention, preferably allyl and/or methallyl bromide and/or chloride and said at least one basic catalyst is suitably and preferably at least one alkali and/or alkaline earth metal hydroxide, alkoxide and/or carbonate, such as potassium and/or sodium hydroxide, carbonate and/or methoxide.

Said at least one alcohol, having one or more hydroxyl groups, in step (ii) subjected to reaction with the product obtained in step (i) is most preferably at least one mono, di, tri or polyalcohol, such as at least one alkanol, alkanediol, 2,2-alkyl-1,3-propanediol, 2-alkyl--2-hydroxyalkyl-1,3-propanediol, 2,2-dihydroxyalkyl-1,3-propanediol or at least one dimer, trimer or polymer of a said alcohol. Embodiments of said preferred alcohols include the most preferred species, which include methanol, 2-ethylhexanediol, ethylene glycol, neopentyl glycol, trimethylolpropane and trimethylolethane.

Said at least one optional organic acid catalyst is most preferably p-toluenesulphonic acid and/or methanesulphonic acid.

The most preferred embodiments of the process of the present invention include embodiments wherein said cyclic formal subjected to allylation in step (i) is 5,5-dihydroxymethyl-1,3-dioxane optionally present in a mixture, such as a said waste stream, of by-products as disclosed above and that said alcohol in step (ii) subjected to reaction with in step (i) yielded allyl and/or methallyl ether is methanol or trimethylolpropane.

The process of the present invention can provide an allyl and/or methallyl ether of a tri or polyhydric alcohol. Said allyl and/or methallyl ether is in the most preferred embodiments a monoallyl, diallyl, monomethallyl and/or dimethallyl ether of pentaerythritol and most preferably obtained from 5,5-dihydroxymethyl-1,3-dioxane recovered from or present in a mixture of by-products, such as a waste stream, yielded in a pentaerythritol synthesis.

In the following Examples 1-4 show embodiments of step (i) of the present process, Examples 5 and 6 show embodiments of step (ii) of the present process, Example 7 shown an embodiment of the optional intermediate purification step and Example 8 shows an embodiment of the optional final purification step.

### EXAMPLE 1

A mixture of polyhydric compounds (Polyol PX™, Perstorp Specialty Chemicals AB, Sweden) obtained from a pentaerythritol synthesis was in an amount comprising 853 g (4 moles) of the cyclic pentaerythritol formal 5,5-hydroxymethyl-1,3-dioxane subjected to reaction with 309 g (4 moles) of allyl chloride in the presence of 352 g (4.4 moles) of sodium hydroxide. The reaction was performed in a 2000 ml 3-necked reaction flask equipped with a thermometer, condenser, Dean-Stark receiver, stirrer and a drop funnel. The reaction was under stirring carried out at a temperature of 120°C. The reaction time was 7 hours. Yielded reaction water was continuously collected in the Dean-Stark receiver. Obtained reaction mixture was neutralised with hydrochloric acid to a pH of 7. Volatile by-products were removed by evaporation at 60°C and 1330 Pa (10 mm Hg) and formed sodium chloride was removed by filtration.

877 g of the reaction mixture was recovered and GC analyses showed that a 61 % selectivity of monallyl ether of cyclic pentaerythritol formal was obtained with a 56% conversion of cyclic pentaerythritol formal.

### EXAMPLE 2

Example 1 was repeated with the difference that Polyol PX™ in an amount comprising 326 g (1.53 mole) of CPF was subjected to reaction with 296 g (3.83 moles) of allyl chloride and 337 g (4.21 moles) of sodium hydroxide.

390 g of the reaction mixture was recovered and GC analyses showed that a 50% selectivity of monallyl ether of cyclic pentaerythritol formal was obtained with a 100% conversion of cyclic pentaerythritol formal.

### EXAMPLE 3

Example 1 was repeated with the difference that Polyol PX™ in an amount comprising 759 g (4.97 moles) of CPF was subjected to reaction with 384 g (4.97 moles) of allyl chloride and 437 g (5.46 moles) of sodium hydroxide.

895 g of the reaction mixture was recovered and GC analyses showed that a 82% selectivity of monallyl ether of cyclic pentaerythritol formal was obtained with a 72% conversion of cyclic pentaerythritol formal.

### EXAMPLE 4

Example 1 was repeated with the difference that Polyol PX™ in an amount comprising 1128 g (7.4 moles) of CPF was subjected to reaction with 687 g (8.88 moles) of allyl chloride and 710 g (8.88 moles) of sodium hydroxide.

1426 g of the reaction mixture was recovered and GC analyses showed that a 87% selectivity of monallyl ether of cyclic pentaerythritol formal was obtained with a 88% conversion of cyclic pentaerythritol formal.

### EXAMPLE 5

400 g of a reaction mixture, comprising 1.32 mole of monoallyl ether of cyclic pentaerythritol formal, obtained in accordance with Examples 1-4, was subjected to reaction with 385 g (2.81 moles) of trimethylolpropane in the presence of 4.9 g (0.05 mole) of p-toluenesulphonic acid. The reaction was performed in a 2000 ml 3-necked reaction flask equipped with a thermometer, stirrer and a distillation column with a Sulzer packing. The reaction was under stirring carried out at a temperature of 135°C and a pressure of 133 Pa (1 mm Hg). The reaction time was 6 hours.

1219 g of reaction mixture was recovered and GC analyses showed that a 95% selectivity of pentaerythritol monoallyl ether was obtained with a 87% conversion of monoallyl ether of cyclic pentaerythritol formal.

### EXAMPLE 6

1500 g of a reaction mixture, comprising 7.91 moles of monoallyl ether of cyclic pentaerythritol formal, obtained in accordance with Examples 1-4, was subjected to reaction with 2105 g (65.7 moles) of methanol in the presence of 15.0 g (0.079 mole) of p-toluenesulphonic acid. The reaction was performed in a 3-necked reaction flask equipped with a thermometer, stirrer and a distillation column with a Sulzer packing was. The reaction was under stirring carried out at a temperature of 85°C and a pressure of 1013 hPa (760 mm Hg). The reaction time was 72 hours.

### EXAMPLE 7

1893 g of a reaction admixture, obtained in accordance with Examples 1-4, comprising 93.3% monoallyl ether and 5.3 % diallyl ether of cyclic pentaerythritol formal was purified in a batch distillation equipment at a pressure of 133 Pa (1 mm Hg). The distillation column was packed with a Sulzer BX packing. The reflux was 10:1 at the beginning of the distillation and 5:1 at the end.

1298 g of the main fraction was recovered. The distillation yield was 73% and GC analyses showed a 99% purity of monoallyl ether of cyclic pentaerythritol formal.

### EXAMPLE 8

1398 g of a reaction admixture, obtained in accordance with Examples 5 and 6, comprising 89.9% pentaerythritol monoallyl ether was purified in a batch distillation equipment at a pressure of 133 Pa (1 mm Hg). The column was packed with a Sulzer BX packing. The reflux was 10:1.

1113 g of the main fraction was recovered. The distillation yield was 87% and GC analyses showed 98% purity of pentaerythritol monoallyl ether.

## Claims

1. A process for production of an allyl and/or methallyl ether of a tri or polyhydric alcohol c h a r a c t e r i s e d i n, that said process comprises the steps of
i) subjecting at least one cyclic formal of at least one tri or polyhydric alcohol to allylation by reaction with at least one allyl and/or methallyl halide in presence of a catalytically effective amount of at least one basic catalyst, whereby a reaction mixture, comprising at least one allyl and/or methallyl ether of said cyclic formal, is yielded, and
ii) subjecting in step (i) yielded allyl and/or methallyl ether of said cyclic formal to reaction with at least one alcohol, having one or more hydroxyl groups, optionally in presence of a catalytically effective amount of at least one organic acid catalyst, whereby a reaction mixture, comprising at least one allyl and/or methallyl ether of said tri or polyhydric alcohol and at least one formal of said alcohol, is yielded
and optionally an intermediate purification step wherein the reaction mixture obtained in step (i) is purified prior to initiation of step (ii) and/or a final purification step wherein the reaction mixture obtained in step (ii) is purified.

2. A process according to Claim 1**characterised in, that** said step (i) is performed at a temperature of 60-140°C.

3. A process according to Claim 1 or 2 **characterised in, that** said step (ii) is performed at a temperature of 80-160°C.

4. A process according to any of the Claims 1-3 **characterised in, that** said optional intermediate purification comprises extraction and optionally further purification by evaporation, such as distillation.

5. A process according to any of the Claims 1-4 **characterised in, that** said optional final purification step comprises purification of the reaction mixture obtained in step (ii) by evaporation, such as distillation.

6. A process according to any of the Claims 1-5 **characterised in, that** said at least one cyclic formal is a recovered by-product or is present in a mixture of by-products from a synthesis of a tri or polyalcohol.

7. A process according to any of the Claims 1-5 **characterised in, that** said at least one cyclic formal is recovered from a waste stream and/or a mixture of by-products from a synthesis of a tri or polyalcohol and optionally that said cyclic formal is purified.

8. A process according to any of the Claims 1-7 **characterised in, that** said at least one cyclic formal is at least one cyclic formal of a 1,2,3-propanetriol, 2-alkyl-2-hydroxyalkyl-1,3-propanediol, 2-alkyl-2-hydroxyalkoxy-1,3-propandiol, 2-alkyl-2-hydroxyalkoxyalkyl-1,3-propanediol, 2,2-dihydroxyalkyl-1,3-propanediol, 2,2-dihydroxyalkoxy-1,3-propanediol or 2,2-dihydroxyalkoxyalkyl-1,3-propanediol.

9. A process according to any of the Claims 1-7**characterised in, that** said at least one cyclic formal is at least one cyclic formal of at least one dimer, trimer or polymer of a 1,2,3-propanetriol, 2-alkyl-2-hydroxyalkyl-1,3-propanediol, 2-alkyl-2-hydroxyalkoxy-1,3-propandiol, 2-alkyl-2-hydroxyalkoxyalkyl-1,3-propanediol, 2,2-dihydroxyalkyl-1,3-propanediol, 2,2-dihydroxyalkoxy-1,3-propanediol or 2,2-dihydroxyalkoxyalkyl-1,3-propanediol.

10. A process according to any of the Claims 1-9 **characterised in, that** said at least one cyclic formal is at least one cyclic formal of glycerol, trimethylolethane, trimethylolpropane, diglycerol, ditrimethylolethane, ditrimethylolpropane, pentaerythritol or dipentaerythritol.

11. A process according to any of the Claims 1-9 **characterised in, that** said at least one cyclic formal is at least one cyclic formal of an ethoxylated and/or propoxylated glycerol, trimethylolethane, trimethylolpropane, diglycerol, ditrimethylolethane, ditrimethylolpropane, pentaerythritol or dipentaerythritol.

12. A process according to any of the Claims 1-9 **characterised in, that** said at least one cyclic formal is at least one 4-hydroxyalkyl-1,3-dioxolane, 5-hydroxy-1,3-dioxane, 5-alkyl-5-hydroxy-1,3-dioxane, 5-alkyl-5-hydroxyalkyl-1,3-dioxane or 5,5-hydroxyalkyl-1,3-dioxane.

13. A process according to Claim 12 **characterised in, that** said at least one cyclic formal is 5-hydroxy-1,3-dioxane, 5-methyl-5-hydroxymethyl-1,3-dioxane, 5-ethyl-5-hydroxymethyl-1,3-dioxane or 5,5-dihydroxymethyl-1,3-dioxane.

14. A process according to any of the Claims 1-13 **characterised in, that** said at least one allyl and/or methallyl halide is allyl and/or methallyl bromide and/or chloride.

15. A process according to any of the Claims 1-14 **characterised in, that** said at least one basic catalyst is at least one alkali and/or alkaline earth metal hydroxide, alkoxide and/or carbonate.

16. A process according to Claim 15 **characterised in, that** said at least one basic catalyst is potassium and/or sodium hydroxide, carbonate and/or methoxide.

17. A process according to any of the Claims 1-16 **characterised in, that** said at least one alcohol, having one or more hydroxyl groups, is at least one mono, di, tri or polyalcohol.

18. A process according to Claim 17 **characterised in, that** said at least one mono, di, tri or polyalcohol is an alkanol, an alkanediol, a 2,2-alkyl-1,3-propanediol, a 2-alkyl-2-hydroxyalkyl-1,3-propanediol, a 2,2-dihydroxyalkyl-1,3-propanediol or a dimer, trimer or polymer of a said alcohol.

19. A process according to Claim 17 or 18 **characterised in, that** said at least one mono, di, tri or polyalcohol is methanol, 2-ethylhexanediol, ethylene glycol, neopentyl glycol, trimethylolpropane and/or trimethylolethane.

20. A process according to any of the Claims 1-19 **characterised in, that** said at least one organic acid catalyst is p-toluenesulphonic acid and/or methanesulphonic acid.

21. A process according to any of the Claims 1-20 **characterised in, that** said at least one cyclic formal subjected to allylation in step (i) is 5,5-dihydroxymethyl-1,3-dioxane or a mixture, such as a waste stream, comprising 5,5-dihydroxymethyl-1,3-dioxane and that said alcohol, which in step (ii) is subjected to reaction with in step (i) yielded allyl and/or methallyl ether, is trimethylolpropane.

## Patentansprüche

1. Verfahren zur Herstellung eines Allyl- und/oder Methallylethers eines drei- oder mehrwertigen Alkohols, **dadurch gekennzeichnet, dass** das Verfahren die Schritte umfasst:
(i) Unterwerfen mindestens eines cyclischen Formals mindestens eines drei- oder mehrwertigen Alkohols einer Allylierung durch Umsetzen mit mindestens einem Allyl- und/oder Methallylhalogenid in Gegenwart einer katalytisch wirksamen Menge mindestens eines basischen Katalysators, wodurch eine Reaktionsmischung, umfassend mindestens einen Allyl - und/oder Methallylether des cyclischen Formals, erhalten wird, und
(ii) Unterwerfen des in Schritt (i) erhaltenen Allyl- und/oder Methallylethers des cyclischen Formals einer Umsetzung mit mindestens einem Alkohol, der eine oder mehrere Hydroxylgruppe(n) hat, gegebenenfalls in Gegenwart einer katalytisch wirksamen Menge mindestens eines organischen Säurekatalysators, wodurch die Reaktionsmischung, umfassend mindestens einen Allyl- und/oder Methallylether des drei- oder mehrwertigen Alkohols und mindestens ein Formal des Alkohols, erhalten wird,
und gegebenenfalls einen Zwischenreinigungsschritt, worin die in Schritt (i) erhaltene Reaktionsmischung vor Einleitung des Schrittes (ii) gereinigt wird, und/oder einen Endreinigungsschritt, worin die in Schritt (ii) erhaltene Reaktionsmischung gereinigt wird.

2. Verfahren gemäss Anspruch 1, **dadurch gekennzeichnet, dass** der Schritt (i) bei einer Temperatur von 60 bis 140°C durchgeführt wird.

3. Verfahren gemäss Anspruch 1 oder 2, **dadurch gekennzeichnet, dass** der Schritt (ii) bei einer Temperatur von 80 bis 160°C durchgeführt wird.

4. Verfahren gemäss irgendeinem der Ansprüche 1 bis 3, **dadurch gekennzeichnet, dass** die optionale Zwischenreinigung eine Extraktion und gegebenenfalls eine weitere Reinigung durch Verdampfen, wie etwa Destillation, umfasst.

5. Verfahren gemäss irgendeinem der Ansprüche 1 bis 4, **dadurch gekennzeichnet, dass** der optionale Endreinigungsschritt eine Reinigung der in Schritt (ii) erhaltenen Reaktionsmischung durch Verdampfen, wie Destillation, umfasst.

6. Verfahren gemäss irgendeinem der Ansprüche 1 bis 5, **dadurch gekennzeichnet, dass** das mindestens eine cyclische Formal ein zurückgewonnenes Nebenprodukt ist oder in einer Mischung von Nebenprodukten aus der Synthese eines drei- oder mehrwertigen Alkohols vorliegt.

7. Verfahren gemäss irgendeinem der Ansprüche 1 bis 5, **dadurch gekennzeichnet, dass** das mindestens eine cyclische Formal aus einem Abfallstrom und/oder einer Mischung von Nebenprodukten aus der Synthese eines drei- oder mehrwertigen Alkohols zurückgewonnen und dieses cyclische Formal gegebenenfalls gereinigt wird.

8. Verfahren gemäss irgendeinem der Ansprüche 1 bis 7, **dadurch gekennzeichnet, dass** das mindestens eine cyclische Formal mindestens ein cyclisches Formal von 1,2,3-Propantriol, 2-Alkyl-2-hydroxyalkyl-1,3-propandiol, 2-Alkyl-2-hydroxyalkoxy-1,3-propandiol, 2-Alkyl-2-hydroxyalkoxyalkyl-1,3-propandiol, 2,2-Dihydroxyalkyl-1,3-propandiol, 2,2-Dihydroxyalkoxy-1,3-propandiol oder 2,2-Dihydroxyalkoxyalkyl-1,3-propandiol ist.

9. Verfahren gemäss irgendeinem der Ansprüche 1 bis 7, **dadurch gekennzeichnet, dass** das mindestens eine cyclische Formal mindestens ein cyclisches Formal von mindestens einem Dimer, Trimer oder Polymer von 1,2,3-Propantriol, 2-Alkyl-2-hydroxyalkyl-1,3-propandiol, 2-Alkyl-2-hydroxyalkoxy-1,3-propandiol, 2-Alkyl-2-hydroxyalkoxyalkyl-1,3-propandiol, 2,2-Dihydroxyalkyl-1,3-propandiol, 2,2-Dihydroxyalkoxy-1,3-propandiol oder 2,2-Dihydroxyalkoxyalkyl-1,3-propandiol ist.

10. Verfahren gemäss irgendeinem der Ansprüche 1 bis 9, **dadurch gekennzeichnet, dass** das mindestens eine cyclische Formal mindestens ein cyclisches Formal von Glycerin, Trimethylolethan, Trimethylolpropan, Diglycerin, Ditrimethylolethan, Ditrimethylolpropan, Pentaerythrit oder Dipentaerythrit ist.

11. Verfahren gemäss irgendeinem der Ansprüche 1 bis 9, **dadurch gekennzeichnet, dass** das mindestens eine cyclische Formal mindestens ein cyclisches Formal von ethoxyliertem und/oder propoxyliertem Glycerin, Trimethylolethan, Trimethylolpropan, Diglycerin, Ditrimethylolethan, Ditrimethylolpropan, Pentaerythrit oder Dipentaerythrit ist.

12. Verfahren gemäss irgendeinem der Ansprüche 1 bis 9, **dadurch gekennzeichnet, dass** das mindestens eine cyclische Formal mindestens ein 4-Hydroxyalkyl-1,3-dioxolan, 5-Hydroxy-1,3-dioxan, 5-Alkyl-5-hydroxy-1,3-dioxan, 5-Alkyl-5-hydroxyalkyl-1,3-dioxan oder 5,5-Hydroxyalkyl-1,3-dioxan ist.

13. Verfahren gemäss Anspruch 12, **dadurch gekennzeichnet dass** das mindestens eine cyclische Formal 5-Hydroxy-1,3-dioxan, 5-Methyl-5-hydroxymethyl-1,3-dioxan, 5-Ethyl-5-hydroxymethyl-1,3-dioxan oder 5,5-Dihydroxymethyl-1,3-dioxan ist.

14. Verfahren gemäss irgendeinem der Ansprüche 1 bis 13, **dadurch gekennzeichnet, dass** das mindestens eine Allyl- und/oder Methallylhalogenid Allyl- und/oder Methallylbromid und/oder -chlorid ist.

15. Verfahren gemäss irgendeinem der Ansprüche 1 bis 14, **dadurch gekennzeichnet, dass** der mindestens eine basische Katalysator mindestens ein Alkali- und/oder Erdalkalimetallhydroxid, -alkoxid und/oder -carbonat ist.

16. Verfahren gemäss Anspruch 15, **dadurch gekennzeichnet, dass** der mindestens eine basische Kalium- und/oder Natriumhydroxid, -carbonat und/oder -methoxid ist.

17. Verfahren gemäss irgendeinem der Ansprüche 1 bis 16, **dadurch gekennzeichnet, dass** der mindestens eine Alkohol mit einer oder mehreren Hydroxylgruppen mindestens ein Mono-, Di-, Tri- oder Polyalkohol ist.

18. Verfahren gemäss Anspruch 17, **dadurch gekennzeichnet, dass** der mindestens eine Mono-, Di-, Tri- oder Polyalkohol ein Alkanol, ein Alkandiol, ein 2,2-Alkyl-1,3-propandiol, ein 2-Alkyl-2-hydroxyalkyl-1,3-propandiol, ein 2,2-Dihydroxyalkyl-1,3-propandiol oder ein Dimer, Trimer oder Polymer des Alkohols ist.

19. Verfahren gemäss Anspruch 17 oder 18, **dadurch gekennzeichnet, dass** der mindestens eine Mono-, Di-, Tri- oder Polyalkohol Methanol, 2-Ethylhexandiol, Ethylenglykol, Neopentylglykol, Trimethylolpropan und/oder Trimethylolethan ist.

20. Verfahren gemäss irgendeinem der Ansprüche 1 bis 19, **dadurch gekennzeichnet, dass** der mindestens eine organische Säurekatalysator p-Toluolsulfonsäure und/oder Methansulfonsäure ist.

21. Verfahren gemäss irgendeinem der Ansprüche 1 bis 20, **dadurch gekennzeichnet, dass** das mindestens eine cyclische Formal, das in Schritt (i) einer Allylierung unterworfen wird, 5,5-Dihydroxymethyl-1,3-dioxan oder eine Mischung, wie etwa ein Abfallstrom, der 5,5-Dihydroxymethyl-1,3-dioxan umfasst, ist und dass der Alkohol, der in Schritt (ii) einer Umsetzung mit dem in Schritt (i) erhaltenen Allyl- und/oder Methallylether unterworfen wird, Trimethylolpropan ist.

## Revendications

1. Procédé de production d'un éther allylique et/ou méthallylique d'un alcool tri ou polyhydrique **caractérisé en ce que** ledit procédé comprend les étapes de
i) soumission d'au moins un formal cyclique d'au moins un alcool tri ou polyhydrique à une allylation par réaction avec au moins un halogénure d'allyle et/ou de méthallyle en présence d'une quantité catalytiquement efficace d'au moins un catalyseur basique, d'où il résulte qu'un mélange réactionnel, comprenant au moins un éther allylique et/ou méthallylique dudit formal cyclique, est obtenu, et
ii) soumission de l'éther allylique et/ou méthallylique dudit formal cyclique obtenu à l'étape (i) à une réaction avec au moins un alcool, ayant un ou plusieurs groupe(s) hydroxyle, facultativement en présence d'une quantité catalytiquement efficace d'au moins un catalyseur acide organique, d'où il résulte qu'un mélange réactionnel, comprenant au moins un éther allylique et/ou méthallylique dudit alcool tri ou polyhydrique et au moins un formal dudit alcool, est obtenu
et facultativement une étape de purification intermédiaire dans laquelle le mélange réactionnel obtenu à l'étape (i) est purifié avant l'initiation de l'étape (ii) et/ou une étape de purification finale dans laquelle le mélange réactionnel obtenu à l'étape (ii) est purifié.

2. Procédé selon la revendication 1 **caractérisé en ce que** ladite étape (i) est mise en oeuvre à une température de 60 à 140°C.

3. Procédé selon la revendication 1 ou 2 **caractérisé en ce que** ladite étape (ii) est mise en oeuvre à une température de 80 à 160°C.

4. Procédé selon l'une quelconque des revendications 1 à 3 **caractérisé en ce que** ladite purification intermédiaire facultative comprend une extraction et facultativement une autre purification par évaporation, telle qu'une distillation.

5. Procédé selon l'une quelconque des revendications 1 à 4 **caractérisé en ce que** ladite étape de purification finale facultative comprend une purification du mélange réactionnel obtenu à l'étape (ii) par évaporation, telle qu'une distillation.

6. Procédé selon l'une quelconque des revendications 1 à 5 **caractérisé en ce que** ledit au moins un formal cyclique est un sous-produit récupéré ou est présent dans un mélange de sous-produits provenant d'une synthèse d'un tri ou polyalcool.

7. Procédé selon l'une quelconque des revendications 1 à 5 **caractérisé en ce que** ledit au moins un formal cyclique est récupéré d'un flux de déchets et/ou d'un mélange de sous-produits provenant d'une synthèse d'un tri ou polyalcool et facultativement **en ce que** ledit formal cyclique est purifié.

8. Procédé selon l'une quelconque des revendications 1 à 7 **caractérisé en ce que** ledit au moins un formal cyclique est au moins un formal cyclique d'un 1,2,3-propanetriol, 2-alkyl-2-hydroxyalkyl-1,3-propanediol, 2-alkyl-2-hydroxyalcoxy-1,3-propanediol, 2-alkyl-2-hydroxyalcoxyalkyl-1,3-propanediol, 2,2-dihydroxyalkyl-1,3-propanediol, 2,2-dihydroxyalcoxy-1,3-propanediol, ou 2,2-dihydroxyalcoxyalkyl-1,3-propanediol.

9. Procédé selon l'une quelconque des revendications 1 à 7 **caractérisé en ce que** ledit au moins un formal cyclique est au moins un formal cyclique d'au moins un dimère, trimère ou polymère d'un 1,2,3-propanetriol, 2-alkyl-2-hydroxyalkyl-1,3-propanediol, 2-alkyl-2-hydroxyalcoxy-1,3-propanediol, 2-alkyl-2-hydroxyalcoxyalkyl-1,3-propanediol, 2,2-dihydroxyalkyl-1,3-propanediol, 2,2-dihydroxyalcoxy-1,3-propanediol, ou 2,2-dihydroxyalcoxyalkyl-1,3-propanediol.

10. Procédé selon l'une quelconque des revendications 1 à 9 **caractérisé en ce que** ledit au moins un formal cyclique est au moins un formal cyclique de glycérol, triméthyloléthane, triméthylolpropane, diglycérol, ditriméthyloléthane, ditriméthylolpropane, pentaérythritol ou dipentaérythritol.

11. Procédé selon l'une quelconque des revendications 1 à 9 **caractérisé en ce que** ledit au moins un formal cyclique est au moins un formal cyclique d'un glycérol, triméthyloléthane, triméthylolpropane, diglycérol, ditriméthyloléthane, ditriméthylolpropane, pentaérythritol ou dipentaérythritol éthoxylé et/ou propoxylé.

12. Procédé selon l'une quelconque des revendications 1 à 9 **caractérisé en ce que** ledit au moins un formal cyclique est au moins un 4-hydroxyalkyl-1,3-dioxolane, 5-hydroxy-1,3-dioxane, 5-alkyl-5-hydroxy-1,3-dioxane, 5-alkyl-5-hydroxyalkyl-1,3-dioxane ou 5,5-hydroxyalkyl-1,3-dioxane.

13. Procédé selon la revendication 12 **caractérisé en ce que** ledit au moins un formal cyclique est le 5-hydroxy-1,3-dioxane, 5-méthyl-5-hydroxyméthyl-1,3-dioxane, 5-éthyl-5-hydroxyméthyl-1,3-dioxane ou 5,5-dihydroxyméthyl-1,3-dioxane.

14. Procédé selon l'une quelconque des revendications 1 à 13 **caractérisé en ce que** ledit au moins un halogénure d'allyle et/ou de méthallyle est le bromure et/ou le chlorure d'allyle et/ou de méthallyle.

15. Procédé selon l'une quelconque des revendications 1 à 14 **caractérisé en ce que** ledit au moins un catalyseur basique est au moins un hydroxyde, alcoxyde et/ou carbonate de métal alcalin et/ou alcalinoterreux.

16. Procédé selon la revendication 15 **caractérisé en ce que** ledit au moins un catalyseur basique est l'hydroxyde, carbonate et/ou méthoxyde de potassium et/ou sodium.

17. Procédé selon l'une quelconque des revendications 1 à 16 **caractérisé en ce que** ledit au moins un alcool, ayant un ou plusieurs groupe(s) hydroxyle, est au moins un mono, di, tri ou polyalcool.

18. Procédé selon la revendication 17 **caractérisé en ce que** ledit au moins un mono, di, tri ou polyalcool est un alcanol, un alcanediol, un 2,2-alkyl-1,3-propanediol, un 2-alkyl-2-hydroxyalkyl-1,3-propanediol, un 2,2-dihydroxyalkyl-1,3-propanediol ou un dimère, trimère ou polymère d'un dit alcool.

19. Procédé selon la revendication 17 ou 18 **caractérisé en ce que** ledit au moins un mono, di, tri ou polyalcool est le méthanol, le 2-éthylhexanediol, l'éthylène glycol, le néopentyle glycol, le triméthylolpropane et/ou le triméthyloléthane.

20. Procédé selon l'une quelconque des revendications 1 à 19 **caractérisé en ce que** ledit au moins un catalyseur acide organique est l'acide p-toluènesulfonique et/ou l'acide méthanesulfonique.

21. Procédé selon l'une quelconque des revendications 1 à 20 **caractérisé en ce que** ledit au moins un formal cyclique soumis à une allylation à l'étape (i) est le 5,5-dihydroxyméthyl-1,3-dioxane ou un mélange, tel qu'un flux de déchets, comprenant du 5,5-dihydroxyméthyl-1,3-dioxane et **en ce que** ledit alcool, qui à l'étape (ii) est soumis à une réaction avec l'éther allylique et/ou méthallylique obtenu à l'étape (i), est le triméthylolpropane.
